(19) Europäisches Patentamt
European Patent Office
Office européen des brevets

(11) Veröffentlichungsnummer: **0 413 259 A2**

# (12) EUROPÄISCHE PATENTANMELDUNG

(21) Anmeldenummer: **90115304.9**

(51) Int. Cl.[5]: **C07C 209/82**

(22) Anmeldetag: **09.08.90**

(30) Priorität: **12.08.89 DE 3926765**

(43) Veröffentlichungstag der Anmeldung: **20.02.91 Patentblatt 91/08**

(84) Benannte Vertragsstaaten: **AT BE CH DE DK ES FR GB IT LI NL SE**

(71) Anmelder: **HOECHST AKTIENGESELLSCHAFT
Postfach 80 03 20
D-6230 Frankfurt am Main 80(DE)**

(72) Erfinder: **Fruth, Anton
Edhofer Strasse 6
D-8333 Hebertsfelden(DE)**

## (54) Verfahren zur Verminderung von primärem und sekundärem Amin in einem tertiären Amin.

(57) Es wird ein Verfahren zur Verminderung des Gehaltes an primärem und sekundärem Amin in einem tertiären Amin beschrieben, wobei das zu behandelnde tertiäre Amin mit einem organischen Anhydrid in Kontakt gebracht wird, bis die angestrebte Verminderung des Gehaltes an primärem und sekundärem Amin erreicht ist. Durch das genannte Inkontaktbringen, das in der Regel bei einer Temperatur von 20 bis 200 °C durchgeführt wird, entstehen aus dem primären Amin und dem sekundären Amin, das besonders unerwünscht ist, die Verbindungen Säure und/oder Säureamid. Nach Abtrennung dieser Verbindungen, die vergleichsweise unschädlich sind, liegt ein vollständig reines tertiäres Amin vor.

EP 0 413 259 A2

# VERFAHREN ZUR VERMINDERUNG VON PRIMÄREM UND SEKUNDÄREM AMIN IN EINEM TERTIÄREN AMIN

Die Erfindung betrifft ein Verfahren zur Verminderung des Gehaltes an primärem und sekundärem Amin in einem tertiären Amin.

Tertiäre Amine enthalten bekanntlich aufgrund ihrer Herstellungsweise im allgemeinen eine mehr oder weniger große Menge an primärem und sekundärem Amin. Dies gilt insbesondere für solche tertiäre Aminprodukte, die durch das sehr häufig verwendete Aminolyse-Verfahren hergestellt worden sind, das heißt durch Umsetzung von sekundären Aminen mit Alkoholen als Alkylierungsmittel in Gegenwart von Dehydrierungs-/Hydrierungskatalysatoren, vergleiche US-Patentschrift 4 138 437, insbesondere Beispiel 1, Zeilen 46 bis 48 und britische Patentschrift 1 585 480, insbesondere Spalte 1, Zeilen 54 bis 59. Das nach diesem an sich vorteilhaften Verfahren erhaltene tertiäre Amin hat in der Regel einen Gehalt an primärem und/oder sekundärem Amin von insgesamt bis zu etwa 2 Gew.-Teilen pro 100 Gew.-Teile tertiäres Amin, wobei das sekundäre Amin im Vergleich zum primären die Hauptmenge darstellt (beim primären Amin handelt es sich in der Regel lediglich um Spuren). Gerade die sekundären Amine sind aber eine besonders unerwünschte Verunreinigung, weil aus diesen Aminen bekanntlich Nitrosamine entstehen können. Die primären und sekundären Amine, insbesondere in Form der sogenannten Methylalkylamine, sind wegen der geringen Siedepunktdifferenz oft destillativ nicht trennbar. Dies gilt insbesondere dann, wenn es sich um sogenannte Kettenverschnitte handelt (was in der Regel der Fall ist), bei denen sich die Siedepunkte der Mono- und Dimethylalkylamine sogar überschneiden.

Die Aufgabe der Erfindung besteht demnach darin, ein Verfahren anzugeben, mit dem eine weitgehende bis vollständige Minderung der in Rede stehenden Verunreinigungen möglich ist und damit ein von primären und sekundären Aminen praktisch freies tertiäres Amin erreicht werden kann. Das neue Verfahren soll ferner wenig aufwendig und leicht durchzuführen sein.

Das erfindungsgemäße Verfahren zur Verminderung des Gehaltes an primärem und sekundärem Amin in einem tertiären Amin ist dadurch gekennzeichnet, daß das zu behandelnde tertiäre Amin mit einem organischen Anhydrid in Kontakt gebracht wird, bis die angestrebte Verminderung des Gehaltes an primärem und sekundärem Amin erreicht ist.

Die dem erfindungsgemäßen Reinigungsverfahren zugrundeliegende chemische Reaktion dürfte gemäß nachstehenden Gleichungen ablaufen. Die Gleichungen enthalten als organisches Anhydrid Essigsäureanhydrid, Bernsteinsäureanhydrid und Phthalsäureanhydrid und als primäres und sekundäres Amin (das sind die umzusetzenden Amine) Dodecylamin und Methyldodecylamin:

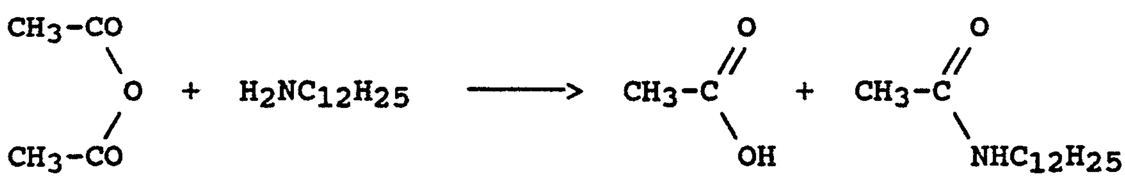

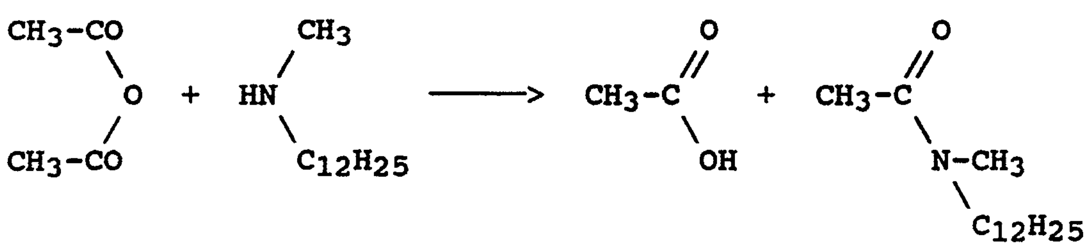

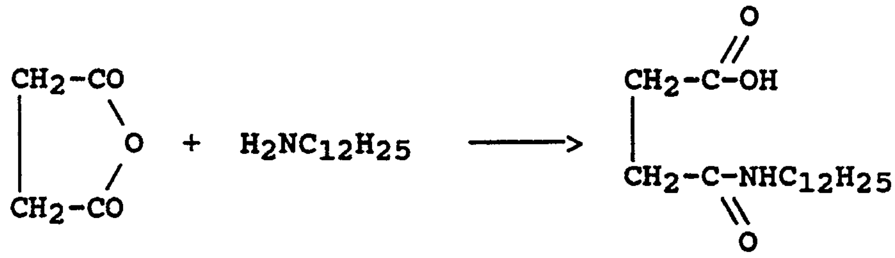

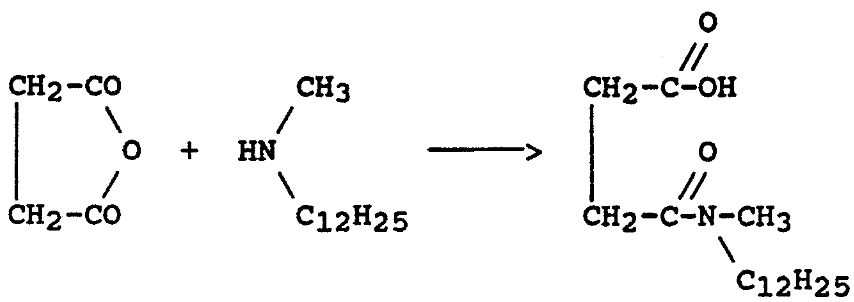

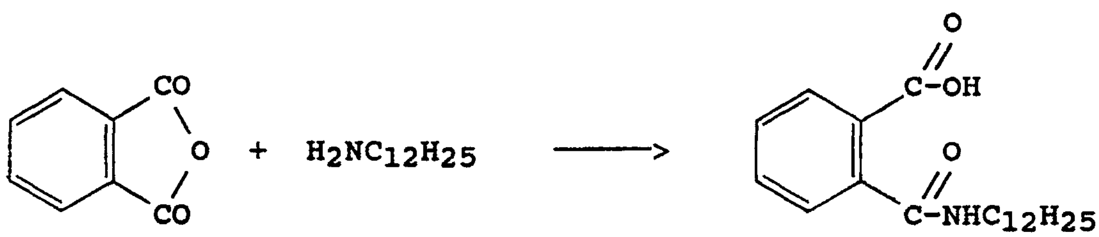

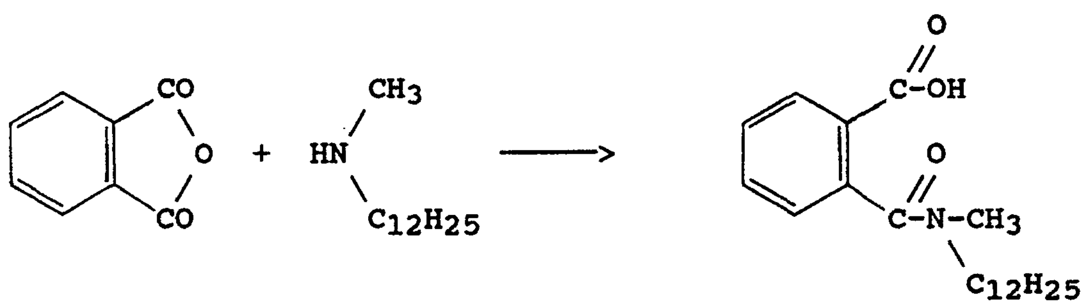

Wie die Reaktionsgleichungen zeigen, entsteht aus dem unerwünschten primären und sekundären Amin und dem eingesetzten Anhydrid Säure und Säureamid oder Amidsäure (im folgenden ebenfalls als Säureamid bezeichnet). Die im tertiären Amin nun vorliegenden Komponenten, Säure und/oder Säureamid, sind entweder abtrennbar, beispielsweise im Rahmen einer Destillation, oder sie können im tertiären Amin verbleiben, da daraus keine nennenswerten Nachteile resultieren, was insbesondere für das Säureamid gilt.

Aufgrund der hohen Reaktionsfähigkeit der Anhydride läuft die erfindungsgemäße Reaktion oft auch schon bei niedrigen Temperaturen ab, so daß bereits ein bloßes Inkontaktbringen des zu behandelnden tertiären Amins mit dem Anhydrid die angestrebte Umwandlung bringt. Andererseits können auch höhere Temperaturen angewandt werden. Eine höhere Temperatur ist dann zweckmäßig, wenn das zu behandelnde tertiäre Aminprodukt einen relativ hohen Schmelzpunkt besitzt und/oder wenn eine besonders kurze Reaktionszeit gewünscht wird. Das erfindungsgemäße Verfahren wird demnach in der Regel bei einer Temperatur von 20 bis 200 °C durchgeführt, vorzugsweise bei einer Temperatur von 50 bis 150 °C. Die Reaktionszeit, das ist die Zeit, die für die Umsetzung des Anhydrids mit dem primären und/oder sekundären Amin benötigt wird, beträgt in der Regel 0,3 bis 3 Stunden. Sie hängt klarerweise insbesondere

von der Umsetzungstemperatur ab und davon, welcher Umsetzungsgrad angestrebt wird.

Was die Menge an Anhydrid betrifft, hat es sich als vorteilhaft erwiesen, es in einem zumindest äquimolaren Verhältnis zum anwesenden primären und/oder sekundären Amin einzusetzen. So beträgt die Anhydridmenge vorzugsweise 1 bis 2 mol und insbesondere 1,05 bis 1,5 mol pro mol umzusetzendem Amin. Die Menge an primärem und sekundärem Amin im zu behandelnden tertiären Amin kann zum Beispiel durch die bekannte Umsetzung des tertiären Aminproduktes mit Schwefelkohlenstoff und Titration der aus dem primären und sekundären Amin gebildeten Thiosäuren ermittelt werden.

Die Art des erfindungsgemäß einzusetzenden organischen Anhydrids ist an sich nicht kritisch. Es versteht sich von selbst, daß man ein auf das zu behandelnde tertiäre Amin abgestimmte Anhydrid einsetzen wird. So kommen als Anhydride aliphatische, cycloaliphatische oder aromatische Säureanhydride in Betracht. Bevorzugte Säureanhydride sind solche der nachstehenden Formel

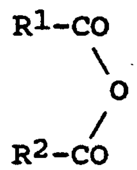

worin bedeuten $R^1$ und $R^2$ einen Alkylrest mit 1 bis 18 C-Atomen, vorzugsweise 2 bis 4 C-Atomen, oder einen Alkenylrest mit 2 bis 18 C-Atomen, vorzugsweise 2 bis 4 C-Atomen, oder $R^1$ und $R^2$ zusammen einen Alkylenrest mit 1 bis 10 C-Atomen, vorzugsweise 2 bis 4 C-Atomen, der auch Doppelbindungen enthalten kann, oder einen gegebenenfalls substituierten ortho-Phenylenrest. Bevorzugte Säureanhydride sind also jene der Monocarbonsäuren, vorzugsweise gesättigt (das heißt $R^1$ und $R^2$ sind gleich und bedeuten den genannten Alkylrest), wie Essigsäureanhydrid, Propionsäureanhydrid, Buttersäureanhydrid und dergleichen, ferner jene der gesättigten oder ungesättigten Dicarbonsäuren (das heißt $R^1$ und $R^2$ stellen zusammen den genannten zweiwertigen Rest dar), wie Malonsäureanhydrid, Bernsteinsäureanhydrid, Adipinsäureanhydrid, Maleinsäureanhydrid und dergleichen, und Phthalsäureanhydrid. Besonders bevorzugte Säureanhydride sind, insbesondere aus praktischen Gründen, Essigsäureanhydrid und Phthalsäureanhydrid.

Nach einer bevorzugten Ausführungsform wird das erfindungsgemäße Verfahren in der Weise durchgeführt, daß man das zu behandelnde tertiäre Amin mit dem Säureanhydrid in einer mindestens stöchiometrischen (äquimolaren) Menge, bezogen auf anwesendes primäres und sekundäres Amin (vergleiche obige Reaktionsgleichungen), vorzugsweise in einer Menge von 1 bis 2 mol, insbesondere 1,05 bis 1,5 mol, pro mol primäres und sekundäres Amin, versetzt und die Mischung unter Rühren auf eine Temperatur von 20 bis 200 °C, vorzugsweise 50 bis 150 °C, erhitzt und bei dieser Temperatur ebenfalls unter Rühren hält, bis das anwesende primäre und sekundäre Amin den angestrebten niederen Grenzwert erreicht hat oder vollständig umgewandelt ist. Die Umsetzung kann drucklos oder unter Druck durchgeführt werden. Die Abtrennung von überschüssigem Säureanhydrid und von den entstandenen Komponenten, Säure und Säureamid, kann, sofern sie gewünscht wird, beispielsweise auf destillativem Wege erreicht werden. Säure und Säureanhydrid können zum Beispiel auch in der Weise vom tertiären Amin abgetrennt werden, daß das Reaktionsgemisch mit Alkali (fest oder in Form einer wäßrigen Lösung) behandelt wird zur Umwandlung der Säure und des Säureanhydrids in die entsprechenden Alkalimetallsalze. Von dem nun vorliegenden Gemisch kann das tertiäre Amin ohne weiteres als Destillat gewonnen werden, während die genannten Metallsalze im Sumpf verbleiben. Wie bereits oben erwähnt, resultieren aus dem anwesenden Säureamid im tertiären Amin in der Regel keine besonderen Nachteile.

Bei den tertiären Aminen im Rahmen der vorliegenden Erfindung handelt es sich bevorzugt um solche der nachstehenden Formel

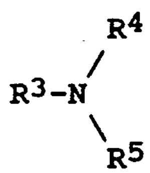

worin bedeuten $R^3$ einen Alkylrest oder einen Alkenylrest mit 6 bis 24 C-Atomen, vorzugsweise 8 bis 22 C-Atomen, und $R^4$ und $R^5$, die vorzugsweise gleich sind, einen Alkylrest mit 1 bis 4 C-Atomen, vorzugsweise den Methylrest. Als Beispiele für $R^3$ seien genannt Octyl, Dodecyl, Tetradecyl, Stearyl, Oleyl, Talgfettalkyl

und Cocosalkyl. Im folgenden sei noch die eingangs erwähnte Aminolyse in Form einer Reaktionsgleichung dargestellt, wobei als Alkohol Dodecylalkohol (Laurylalkohol) und als Amin Dimethylamin fungieren:

```
                          CH3                          CH3
                         /                            /
C12H25-OH   +   H-N                ------>   C12H25-N        +    H2O
                         \                            \
                          CH3                          CH3
```

Neben dieser Hauptreaktion erfolgt bekanntlich eine Disproportionierung des Dimethylamins zu unter anderem Monomethylamin, das mit dem anwesenden Alkohol das sekundäre Amin Methyldodecylamin bildet. Die in Rede stehenden unerwünschten sekundären Amine entsprechen demnach im allgemeinen der Formel $R^3$-NH-$R^4$, wobei $R^3$ und $R^4$ die angegebenen Bedeutungen haben.

Das erfindungsgemäße Verfahren weist eine Reihe von Vorteilen auf. Es stellt eine wenig aufwendige und einfache Methode zur partiellen bis vollständigen Eliminierung von primärem und sekundärem Amin in einem tertiären Amin dar, wobei es ohne weiteres möglich ist, ein tertiätes Amin zu erhalten, das praktisch frei ist von den genannten Verunreinigungen. Das beim erfindungsgemäßen Verfahren angewandte Reagenz, nämlich Säureanhydrid, ist im allgemeinen leicht erhältlich und von hoher Reaktivität. Dadurch kann schon bei relativ milden Reaktionsbedingungen eine vollständige Umsetzung erreicht werden. Die bei der Umsetzung entstehenden Umwandlungsprodukte sind, sofern sie überhaupt stören, in der Regel ohne weiteres abtrennbar, so daß ein nicht nur von primären und sekundären Aminen, sondern auch von anderen Verbindungen freies und damit vollständig reines tertiäres Amin erreichbar ist.

Die Erfindung wird nun an Beispielen noch näher erläutert.

Beispiel 1

In einem mit Rührer, Rückflußkühler und Thermometer ausgestatteten Reaktionsgefäß wurden 100 g zu behandelndes tertiäres Amin der Formel $C_{12-14}$-N$(CH_3)_2$, die 1,8 g (0,008 mol) sekundäres Amin der Formel $C_{12-14}$-NH-$CH_3$ enthielten, eingebracht und mit 0,83 g (0,008 mol) Essigsäureanhydrid versetzt. Die Mischung wurde unter Rühren auf 100 °C erhitzt und bei dieser Temperatur eine Stunde lang gehalten. Nach Abkühlen des Reaktionsgemisches wurde der Gehalt an sekundärem Amin bestimmt. Er betrug nur noch 0,3 Gew.-Teile in 100 Gew.-Teilen tertiärem Amin.

Beispiele 2 bis 8

Diese Beispiele wurden analog Beispiel 1 durchgeführt. Die zu behandelnden tertiären Amine (es handelt sich, ebenso wie im Beispiel 1, um Dimethylalkylamine) und deren Gehalt an sekundärem Amin, das eingesetzte Säureanhydrid, das Molverhältnis von Säureanhydrid zu sekundärem Amin, die Reaktionstemperatur, die Reaktionszeit und die erreichte Reduzierung des Gehaltes an sekundärem Amin sind in der nachstehenden Tabelle zusammengefaßt, die der Vollständigkeit halber auch das Beispiel 1 enthält. Zur Tabelle sei erläuternd noch folgendes gesagt: In Kolonne 2 ist der Alkylrest des Dimethylalkylamins angegeben und die Werte bezüglich des Gehaltes an sekundärem Amin in der dritten und letzten Kolonne sind Gewichtsprozente, bezogen auf zu behandelndes tertiäres Amin (Rohamin).

Zu den Beispielen sei noch ausgeführt, daß in jenen mit Phthalsäureanhydrid als Reagenz für das sekundäre Amin die Abtrennung des überschüssigen Phthalsäureanhydrids und der gebildeten Phthalamidsäure einfach durch Destillation des Reaktionsgemisches erfolgen kann, wobei aufgrund der Siedepunktdifferenzen das Phthalsäureanhydrid und die Phthalamidsäure im Sumpf verbleiben, während das Kopfprodukt das angestrebte reine tertiäre Amin ist. In den Beispielen mit Essigsäureanhydrid erfolgte die Abtrennung des überschüssigen Anhydrids und der gebildeten Essigsäure in folgender Weise: Das Reaktionsgemisch wurde mit einer 30gew.%igen methanolischen Natriummethylatlösung versetzt, so daß eine Natriummethylatmenge eingebracht wurde, die der anwesenden molaren Menge an Essigsäure und Essigsäureanhydrid entspricht, worauf die Mischung zur vollständigen Umwandlung der Essigsäure und des Essigsäureanhydrids in die entsprechenden Natriumsalze bei 70 °C 0,25 Stunden lang gerührt wurde. Die Vakuumdestillation lieferte als Kopfprodukt das (Acetamid enthaltende) tertiäre Amin, während die genannten Natriumsalze im Sumpf verblieben.

TABELLE

| Beispiel | Dimethylalkylamin | Gehalt an sek. Amin | Anhydrid | Molverhältnis von Anhydrid zu sek. Amin | Reaktionstemperatur (°C) | Reaktionszeit (Std.) | Endwert an sek. Amin |
|---|---|---|---|---|---|---|---|
| 1 | $C_{12-14}$-Alkyl | 1,8 | Essigsäureanh. | 1 : 1 | 100 | 1 | 0,3 |
| 2 | $C_8$-Alkyl | 1,8 | Essigsäureanh. | 1 : 1,5 | 100 | 1 | Null |
| 3 | $C_{14}$-Alkyl | 1,5 | Essigsäureanh. | 1 : 1,2 | 70 | 1 | 0,1 |
| 4 | $C_{18}$-Alkyl | 1,5 | Essigsäureanh. | 1 : 1,1 | 70 | 1,3 | Null |
| 5 | Cocosalkyl | 1,5 | Phthalsäureanh. | 1 : 1,3 | 100 | 1 | Null |
| 6 | Talgfettalkyl | 0,7 | Phthalsäureanh. | 1 : 1,5 | 100 | 1 | Null |
| 7 | $C_{12-14}$-Alkyl | 0,7 | Phthalsäureanh. | 1 : 1,5 | 70 | 1 | Null |
| 8 | $C_{12}$-Alkyl | 0,7 | Phthalsäureanh. | 1 : 1,05 | 70 | 1,5 | Null |

**Ansprüche**

1. Verfahren zur Verminderung des Gehaltes an primärem und sekundärem Amin in einem tertiären Amin, dadurch gekennzeichnet, daß das zu behandelnde tertiäre Amin mit einem organischen Anhydrid in Kontakt gebracht wird, bis die angestrebte Verminderung des Gehaltes an primärem und sekundärem Amin erreicht ist.

2. Verfahren nach Anspruch 1, dadurch gekennzeichnet, daß das Anhydrid in einer Menge von 1 bis 2 mol pro mol primärem und sekundärem Amin eingesetzt wird.

3. Verfahren nach Anspruch 1 oder 2, dadurch gekennzeichnet, daß das zu behandelnde tertiäre Amin und das Anhydrid bei einer Temperatur von 20 bis 200 °C in Kontakt gebracht werden.

4. Verfahren nach Anspruch 1 oder 2, dadurch gekennzeichnet, daß das zu behandelnde tertiäre Amin und das Anhydrid bei einer Temperatur von 50 bis 150 °C in Kontakt gebracht werden.

5. Verfahren nach einem oder mehreren der Ansprüche 1 bis 4, dadurch gekennzeichnet, daß ein Anhydrid der nachstehenden Formel eingesetzt wird:

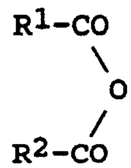

worin bedeuten $R^1$ und $R^2$ einen Alkylrest mit 1 bis 18 C-Atomen oder einen Alkenylrest mit 2 bis 18 C-Atomen oder $R^1$ und $R^2$ zusammen einen Alkylenrest mit 1 bis 10 C-Atomen, der auch Doppelbindungen enthalten kann, oder einen gegebenenfalls substituierten ortho-Phenylenrest.

6. Verfahren nach einem oder mehreren der Ansprüche 1 bis 4, dadurch gekennzeichnet, daß ein Anhydrid der nachstehenden Formel eingesetzt wird:

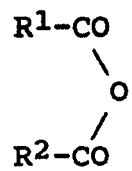

worin bedeuten $R^1$ und $R^2$ einen Alkyl- oder Alkenylrest mit 2 bis 4 C-Atomen oder $R^1$ und $R^2$ zusammen einen Alkylenrest mit 2 bis 4 C-Atomen, der auch Doppelbindungen enthalten kann, oder einen gegebenenfalls substituierten ortho-Phenylenrest.

7. Verfahren nach einem oder mehreren der Ansprüche 1 bis 4, dadurch gekennzeichnet, daß als Anhydrid Essigsäureanhydrid oder Phthalsäureanhydrid eingesetzt wird.